(19) **Europäisches Patentamt / European Patent Office / Office européen des brevets**

(11) **EP 4 566 520 A1**

(12) # EUROPEAN PATENT APPLICATION

(43) Date of publication:
**11.06.2025 Bulletin 2025/24**

(21) Application number: **24213246.2**

(22) Date of filing: **15.11.2024**

(51) International Patent Classification (IPC):
**A61B 5/0205** (2006.01)    **A61B 5/024** (2006.01)
**A61B 5/11** (2006.01)    **A61B 5/145** (2006.01)
**A61B 5/16** (2006.01)    **A61B 5/18** (2006.01)
**A61B 5/00** (2006.01)

(52) Cooperative Patent Classification (CPC):
**A61B 5/1118; A61B 5/02055; A61B 5/02405;
A61B 5/1112; A61B 5/14517; A61B 5/163;
A61B 5/165; A61B 5/18; A61B 5/4845;
A61B 5/6898; A61B 5/746**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **08.12.2023 IN 202321083835**

(71) Applicant: **Tata Consultancy Services Limited
Maharashtra (IN)**

(72) Inventors:
- **BHAVSAR, KARAN RAJESH**
  **400607 Thane, Maharashtra (IN)**
- **LINGAYAT, SUSHRUT SURESH**
  **400601 Thane, Maharashtra (IN)**
- **KIMBAHUNE, SANJAY Madhukar**
  **400021 Mumbai, Maharashtra (IN)**
- **SHINDE, SUJIT RAGHUNATH**
  **400607 Thane, Maharashtra (IN)**
- **VISHWAKARMA, HARSH**
  **400021 Mumbai, Maharashtra (IN)**
- **PAWAR, BHASKAR RAMCHANDRA**
  **400607 Thane, Maharashtra (IN)**

(74) Representative: **Goddar, Heinz J.
Boehmert & Boehmert
Anwaltspartnerschaft mbB
Pettenkoferstrasse 22
80336 München (DE)**

(54) **ALCOHOL INTOXICATION STATE DETECTION USING A WEARABLE DEVICE AND A MOBILE DEVICE**

(57) This disclosure relates generally to a method and system for sensing alcohol intoxication and raising an alert. State-of-the-art methods for detecting alcohol intoxication majorly includes breath alcohol devices, bodily fluid testing, transdermal sensors, mathematical algorithms, and optical techniques. However, the impact of alcohol is not same for all but differs based on individuals' physiological traits and capacity to tolerate. Personalized systems that can sense alcohol intoxication based on individuals present alcohol consumption activity as well as the past history of consumption is not yet achieved. The proposed method of sensing the alcohol intoxication state of the subject is based on cumulative scoring technique using sensors and applications pre-installed in an GPS enabled wearable device and a mobile device. The method also triggers an alert to the subject consuming alcohol as well as to an emergency contact about altered physical and cognitive functioning under the influence of the alcohol.

FIG. 3

**EP 4 566 520 A1**

**Description**

CROSS-REFERENCE TO RELATED APPLICATIONS AND PRIORITY

The present application claims priority from Indian patent application no. 202321083835, filed on December 08, 2023.

TECHNICAL FIELD

The disclosure herein generally relates to automated alcohol intoxication state detection, and, more particularly, to a method and system for sensing the alcohol intoxication state of a subject based on cumulative scoring technique using a pair of global positioning sensing (GPS) enabled wearable device and a mobile device.

BACKGROUND

Every year, millions of people are involved in alcohol related accidents/incidents due to driving and/or working while under the influence of alcohol. Drinking alcohol alters a person's perception and can have a serious effect on the ability to perform a job correctly and safely. This can be very dangerous in various situations, such as driving, operating machinery, and practically any profession in which mistakes can cause serious illness, injury or even death. Regular consumption of excessive amounts of alcohol is also associated with the development of liver disease and increased blood pressure, making those individuals more susceptible to health complications in the future. Alcohol consumption puts a significant burden on public services. Combining the costs of dealing with alcohol-related crime, loss of productivity through unemployment and sickness, and the cost and burden on the health services, the cost of alcohol on society is way too high. Short-term influences of alcohol intoxication, however, do not carry such damaging consequences, yet they are not without harm.

The field of alcohol intoxication sensing is over 100 years old, spanning the fields of medicine, chemistry, and computer science, aiming to produce the most effective and accurate methods of quantifying intoxication levels. Major advancements for sensing the state of alcohol intoxication through quantifying devices and techniques, are estimates, breath alcohol devices, bodily fluid testing, transdermal sensors, mathematical algorithms, and optical techniques. Each of these categories was researched by analyzing their respective performances and drawbacks. Currently, sensing alcohol intoxication techniques include devices of various natures that take advantage of machine learning, optical spectroscopy, and biochemical sensing methods. The optical spectroscopy methods involving ethanol intoxication sensors yielded several results encompassing different aspects of alcohol intoxication, i.e., behavioral, physiological, and chemical changes in the individual's body. The conventional methods found to be effective in sensing alcohol intoxication using one or more pharmacokinetic estimates, breath-sample testing, bodily fluids, physiological changes, transdermal, and optical spectroscopy methods. However, a relatively inexpensive and unobtrusive way to accurately detect whether a person is intoxicated, and preferably to do so without interrupting the person's activities is not available. While many individual methods listed above have proven to be useful in sensing the alcohol intoxication, there remains a great need for coordinated and well-defined integration of the various methods for immediate, effortless, and accurate sensing of the alcohol intoxication and also to facilitate prompt support! rescue in case of emergency caused due to alcohol intoxication.

SUMMARY

Embodiments of the present disclosure present technological improvements as solutions to one or more of the above-mentioned technical problems recognized by the inventors in conventional systems. For example, in one embodiment, a method of intoxication state detection in a subject is provided. The method includes, receiving, a GPS location of a subject from a mobile device carried by the subject. The GPS location is required as an input to the intoxication detection model wherein the correlation is drawn about places the subject visits and the instances of alcohol consumption sensed by the system. The method further includes, acquiring, motion sensing data of the subject comprising gait pattern, activity sensing and hand gestures using inertial motion sensors of the mobile device and a wearable device worn by the subject, wherein the motion sensing data is processed to obtain an alertness score. The alertness detection module receives input from inertial measurement units (IMUs) such as gyroscope, accelerometer and magnetometer about motion, activity, gait pattern and hand gestures and processes it to obtain the alertness score of the subject. The method further includes, acquiring cognitive ability sensing data of the subject from the mobile device based on a response of the subject to a plurality of activities performed on the mobile device, wherein the plurality of activities comprising (i) interacting with an application installed on the mobile device, personalized for the subject, (ii) performing gaze tracking by utilizing a camera of the mobile device, (iii) performing voice and speech analysis by utilizing a microphone of the mobile device, wherein the cognitive ability sensing data is processed to obtain an attentiveness score. The attentiveness detection module processes the cognitive ability sensing data acquired by the mobile phone based on the subject's response to the personalized mobile device application, gaze detection captured though a camera of the mobile device and the speech pattern captured by the microphone. The method further includes, acquiring physiology sensing data from a plurality of sensors on the wearable device and the mobile device, the physiological sensing data comprising (i) body temperature via thermistors, (ii) sweat analysis based on sweat sensing via transdermal sensors, (iii) heart rate variability via Photoplethysmograph (PPG) sensors, wherein the physiology sensing data is processed to obtain a physiology score. The physiology assessment module receives the physiology sensing data through sensors present on the wearable device and the mobile device and processes the signals to generate the physiology score. The method further includes, obtaining a correlation score using a location sensing module wherein the correlation score is derived by a time lapse buffer based on presence of the person on a given GPS location and episodes of alcohol consumption. The method further includes, aggregating the alertness score, the attentiveness score, the physiology score, and the correlation score to obtain an intoxication confidence score of the person. The intoxication confidence score considered a plurality of factors mentioned above which are instrumental in sensing the intoxication state of the subject. The method further includes, alerting, one of the subject and an emergency contact about the intoxication state of the subject in accordance with the intoxication confidence score. The alerts are sent to the subject or to the emergency contact by a plurality of ways wherein the plurality of ways comprising (a) alerting the subject when the intoxication confidence score found within a threshold value; (b) alerting the emergency contact when the intoxication confidence score found to exceed a threshold value; (c) alerting the emergency contact for Save Our Souls (SoS) when the intoxication confidence score is found to exceed the threshold value and an individual scores of the alertness detection module and the physiology assessment module shows abrupt pattern.

In another aspect, a system for an intoxication state detection is provided. The system includes at least one memory storing programmed instructions; one or more Input /Output (I/O) interfaces; and one or more hardware processors, an alertness detection module, an attentiveness detection module, a physiology assessment module, a location sensing module and a fuse and analyze module, operatively coupled to a corresponding at least one memory, wherein the system is configured to receive, via one or more hardware processors, a GPS location of a subject from a mobile device carried by the subject. The GPS location is required as an input to the intoxication detection model wherein the correlation is drawn about places the subject visits and the instances of alcohol consumption. Further, the system is configured to acquire, via the one or more hardware processors, motion sensing data of the subject comprising gait pattern, activity sensing and hand gestures using inertial motion sensors of the mobile device and a wearable device worn by the subject, wherein the motion sensing data is processed to obtain an alertness score. The alertness detection module receives input from inertial measurement units (IMUs) such as gyroscope, accelerometer and magnetometer about motion, activity, gait pattern and hand gestures and processes it to obtain the alertness score of the subject. Further, the system is configured to acquire, via the one or more hardware processors, cognitive ability sensing data of the subject from the mobile device based on a response of the subject to a plurality of activities performed on the mobile device, wherein the plurality of activities comprising (i) interacting with an application installed on the mobile device, personalized for the subject, (ii) performing gaze tracking by utilizing a camera of the mobile device, (iii) performing voice and speech analysis by utilizing a microphone of the mobile device, wherein the cognitive ability sensing data is processed to obtain an attentiveness score. The attentiveness detection module processes the cognitive ability sensing data acquired by the mobile phone based on the subject's response to the personalized mobile device application, gaze detection captured though a camera of the mobile device and the speech pattern captured by the microphone. Further, the system is configured to acquire, via the one or more hardware processors, physiology sensing data from a plurality of sensors on the wearable device and the mobile device, the physiological sensing data comprising (i) body temperature via thermistors, (ii) sweat analysis based on sweat sensing via transdermal sensors, (iii) heart rate variability via Photoplethysmograph (PPG) sensors, wherein the physiology sensing data is processed to obtain a physiology score. The physiology assessment module receives the physiology sensing data through sensors present on the wearable device and the mobile device and processes the signals to generate the physiology score. The system is configured to obtain, via the one or more hardware processors, a correlation score using a location sensing module wherein the correlation score is derived by a time lapse buffer based on presence of the subject on a given GPS location and episodes of alcohol consumption. Further, the system is configured to aggregate, via the one or more hardware processors, the alertness score, the attentiveness score, the physiology score, and the correlation score to obtain an intoxication confidence score of the person. The intoxication confidence score is derived by aggregation of a plurality of factors mentioned above which are instrumental in sensing the intoxication state of the person. Further, the system is configured to alert, via the one or more hardware processors, the subject, and an emergency contact about the intoxication state of the person in accordance with the intoxication confidence score. The alerts are sent to the subject or to the emergency contact by a plurality of ways wherein the plurality of ways comprising (a) alerting the person when the intoxication confidence score found within a threshold value; (b) alerting the emergency contact when the intoxication confidence score found to exceed a threshold value; (c) alerting the emergency contact for Save Our Souls (SoS) when the intoxication confidence score is found to exceed the threshold value and an individual scores of the alertness detection module and the physiology assessment module shows abrupt pattern.

In yet another aspect, a computer program product including a non-transitory computer-readable medium having embodied therein a computer program for alcohol intoxication state detection of the subject is provided. The computer readable program, when executed on a computing device, causes the computing device to receive, via one or more hardware processors, a GPS location of a subject from a mobile device carried by the subject. The GPS location is required as an input to the intoxication detection model wherein the correlation is drawn about places the subject visits and the instances of alcohol consumption. Further, the computer readable program, when executed on a computing device, causes the computing device to acquire, via the one or more hardware processors, motion sensing data of the subject comprising gait pattern, activity sensing and hand gestures using inertial motion sensors of the mobile device and a wearable device worn by the subject, wherein the motion sensing data is processed to obtain an alertness score. The alertness detection module receives input from inertial measurement units (IMUs) such as gyroscope, accelerometer and magnetometer about motion, activity, gait pattern and hand gestures and processes it to obtain the alertness score of the subject. Further, the computer readable program, when executed on a computing device, causes the computing device to acquire, via the one or more hardware processors, cognitive ability sensing data of the subject from the mobile device based on a response of the subject to a plurality of activities performed on the mobile device, wherein the plurality of activities comprising (i) interacting with an application installed on the mobile device, personalized for the subject, (ii) performing gaze tracking by utilizing a camera of the mobile device, (iii) performing voice and speech analysis by utilizing a microphone of the mobile device, wherein the cognitive ability sensing data is processed to obtain an attentiveness score. The attentiveness detection module processes the cognitive ability sensing data acquired by the mobile phone based on the subject's response to the personalized mobile device application, gaze detection captured though a camera of the mobile device and the speech pattern captured by the microphone. Further, the computer readable program, when executed on a computing device, causes the computing device to acquire, via the one or more hardware processors, physiology sensing data from a plurality of sensors on the wearable device and the mobile device, the physiological sensing data comprising (i) body temperature via thermistors, (ii) sweat analysis based on sweat sensing via transdermal sensors, (iii) heart rate variability via Photoplethysmograph (PPG) sensors, wherein the physiology sensing data is processed to obtain a physiology score. The physiology assessment module receives the physiology sensing data through sensors present on the wearable device and the mobile device and processes the signals to generate the physiology score. Further, the computer readable program, when executed on a computing device, causes the computing device to obtain, via the one or more hardware processors, a correlation score using a location sensing module wherein the correlation score is derived by a time lapse buffer based on presence of the person on a given GPS location and episodes of alcohol consumption. Further, the computer readable program, when executed on a computing device, causes the computing device to aggregate, via the one or more hardware processors, the alertness score, the attentiveness score, the physiology score, and the correlation score to obtain an intoxication confidence score of the person. The intoxication confidence score is derived by aggregation of a plurality of factors mentioned above which are instrumental in sensing the intoxication state of the person. Further, the computer readable program, when executed on a computing device, causes the computing device to alert, via the one or more hardware processors, the subject, and an emergency contact about the intoxication state of the subject in accordance with the intoxication confidence score. The alerts are sent to the subject or to the emergency contact by a plurality of ways wherein the plurality of ways comprising (a) alerting the subject when the intoxication confidence score found within a threshold value; (b) alerting the emergency contact when the intoxication confidence score found to exceed a threshold value; (c) alerting the emergency contact for Save Our Souls (SoS) when the intoxication confidence score is found to exceed the threshold value and an individual scores of the alertness detection module and the physiology assessment module shows abrupt pattern.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system 100 for sensing alcohol intoxication, according to some embodiments of the present disclosure. The system 100 includes a cloud server communicating to IOT enabled wearable device 112 worn by a subject carrying a mobile device 114, for example a smart phone.

FIG. 2 illustrates in-built components of the wearable device 112 and the mobile device 114 and their contribution in analysing specific parameters vital to sense alcohol intoxication, according to some embodiments of the present disclosure.

FIG. 3 illustrates a functional block diagram of an intoxication detection model comprising a plurality of modules sensing variation of physiological and physical parameters under the influence of the alcohol.

FIGS. 4A and 4B are flow diagrams of an illustrative method 400 for detection of alcohol intoxication in the subject possessing the wearable device 112 and carrying the mobile device 114, according to some embodiments of the present disclosure.

FIG. 5 is a flow diagram for an on-boarding the mobile device application, according to some embodiments of the present disclosure.

FIG. 6 is a flow diagram of indicating steps for emergency contact verification performed by the method 400, according to some embodiments of the present disclosure

FIG. 7 illustrates mobile device application-based assessment of the subject under the influence of alcohol, according to some embodiments of the present disclosure.

FIG. 8 is a flow diagram or phase-I sensing as performed by the method, according to some embodiments of the present disclosure.

FIG. 9 is a flow diagram for phase-II sensing as performed by the method 400, according to some embodiments of the present disclosure.

FIG. 10 illustrates a plurality of alert mechanism triggered by the intoxication detection model based on scoring mechanism, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

In the foregoing description, terms "person", "people", "user", "subject" and "individual" are used interchangeably, and they refer to a human under the influence of alcohol being sensed for alcohol intoxication by the method of the present disclosure.

Sensing alcohol intoxication generally involves various categories like estimates, breath alcohol devices, bodily fluid testing, transdermal sensors, mathematical algorithms, and optical techniques. Many of the "categories" of ethanol intoxication systems overlap with each other to a varying extent, hence the division of categories is based only on the principal operation of the techniques. Typically, the gold-standard method for measuring blood ethanol levels is through gas chromatography. Early estimation methods based on mathematical equations are largely popular in forensic fields. Breath alcohol devices are the most common type of alcohol sensors on the market and are generally implemented in law enforcement. Transdermal sensors vary largely in their sensing methodologies, but they mostly follow the principle of electrical sensing or enzymatic reaction rate. Optical devices and methodologies perform well, with some cases outperforming breath alcohol devices in terms of the precision of measurement. Other estimation algorithms consider multimodal approaches and should not be considered alcohol sensing devices, but rather as prospective measurement of the intoxication influence. A variety of devices are also used to provide varying levels of intoxication detection. For example, the SCRAM Continuous Alcohol Monitoring device is an ankle-worn, commercial detection device. It is typically used for high-risk, Driving Under the Influence (DUI) alcohol offenders who have been ordered by a court not to consume alcohol. The SCRAM device samples the wearer's perspiration once every 30 minutes in order to measure his BAC levels. In another example, the Kisai Intoxicated LCD Watch, as produced by TokyoFlash, Japan, is a watch that includes a built-in breathalyzer. By breathing into its breathalyzer, the watch detects and displays graphs of the user's blood alcohol content (BAC) level. Additionally, machine learning approaches to detect BAC from data gathered from conventional smartwatches have been used. As smartwatches have been developed, attempts have been made to utilize them to detect alcohol consumption levels. For example, certain conventional approaches have estimated a user's intoxication level using heart rate and temperature detected by a smartwatch worn by the user. Further, certain mobile device applications, such as Intoxicheck (http://intoxicheck.appstor.io) can detect alcohol impairment in users. In use, a user takes a series of reaction, judgment, and memory challenges before and after drinking, which are compared to estimate their intoxication level. Other mobile device applications detect intoxication detection from gait. For example, certain conventional mobile device applications relate to a passive phone-based system that uses the mobile device's accelerometer data to detect whether users had consumed alcohol or not.

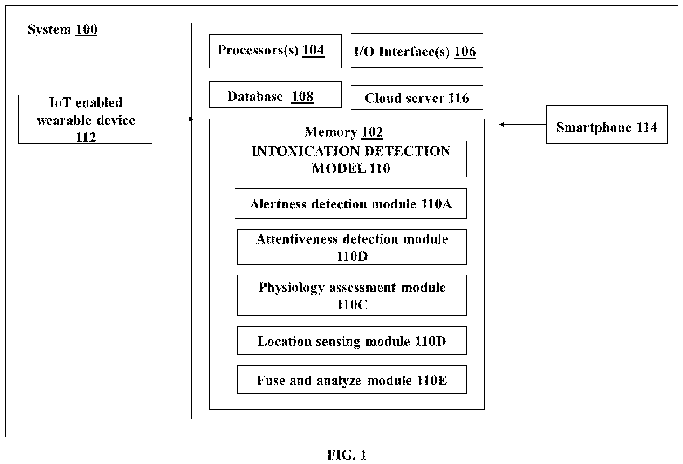

Referring now to the drawings, and more particularly to FIG. 1 through FIG. 10, where similar reference characters denote corresponding features consistently throughout the figures, there are shown preferred embodiments, and these embodiments are described in the context of the following exemplary system and/or method.

FIG. 1 illustrates an exemplary block diagram of a system 100 for sensing alcohol intoxication, according to some embodiments of the present disclosure. The system 100 includes a cloud server communicating to IOT enabled wearable device 112 worn by a subject carrying a mobile device 114, for example a smart phone.

In an embodiment, the cloud server 116 includes one or more processors 104, communication interface device(s) or input/output (I/O) interface(s) 106, and one or more data storage devices or memory 102 operatively coupled to the one or more processors 104. The one or more processors 104 that are hardware processors can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, the system 100 can be implemented in a variety of mobile computing systems, such as wearable devices, mobile devices, laptop computers, notebooks, hand-held devices, workstations, mainframe computers, servers, a network cloud and the like. The I/O interface (s) 106 may include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface(s) 106 can include one or more ports for connecting a number of devices to one another or to another server. The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 102 may include a database or repository. The memory 102 may comprise information pertaining to input(s)/output(s) of each step performed by the processor(s) 104 of the system 100 and methods of the present disclosure. In an embodiment, the database may be external (not shown) to the system 100 and coupled via the I/O interface 106. The memory 102, further include an intoxication detection model 110 which comprises of an alertness detection module 110A, attentiveness detection module 110B, physiology assessment module 110C, location sensing module 110D and fuse & analyze module 110E. The intoxication detection model 110 is a Recurrent Neural Network (RNN) model based on supervised deep learning neural network for modeling the sequence data. The RNN has memory capabilities. It memorizes the output from the previous step, and while making a decision, it takes previous output into consideration to the current input. In the present disclosure, the RNN model is preferred due to its unique ability to work on sequential data. As the usability increases, so as the amount of data gathered by modules, which in turn helps RNN model in building a unique personalized model specific to the user and predicting the user's behavior and facilitating an alert mechanism for alcohol intoxication to the user or to his specified contacts. The IoT enabled wearable device 112, also referred to as wearable device hereinafter, comprises of set of applications and sensors which are relevant for sensing alcohol intoxication in the subjects. Similarly, the mobile device 114 comprises of set of applications and a plurality of sensors which are relevant for sensing alcohol intoxication in the subjects. The wearable device 112 and the mobile device 114 are GPS enabled devices; and a cloud server 116. Sensors and applications of the wearable device 112 and the mobile device 114 are utilized in obtaining input to the system 100 as well as to communicate the output to the subject. The cloud server 116 is utilized in processing the input received from the wearable device 112 and the mobile device 114 and sensing the intoxication state of the subject by scheming an intoxication confidence score based on which a plurality of alerts is triggered about the intoxication state. In the memory, 102, the alertness detection module 110A is functionally connected to a wearable device and a mobile device to receive the signals and processing the signals in the form of an output relevant for sensing whether a subject (i.e. the person) is under the influence of alcohol or not. The attentive detection module 110B is functionally connected to the wearable device and the mobile device to receive the signals and processing the signals in the form of an output relevant for sensing whether a subject (i.e. the person) is under the influence of alcohol or not. The physiology assessment module 110C is functionally connected to the wearable device and the mobile device to receive the signals and processing the signals in the form of an output relevant for sensing whether a subject (i.e. the person) is under the influence of alcohol or not. The location sensing module 110D is functionally connected to the wearable device and the mobile device to receive the signals and processing the signals in the form of an output relevant for sensing the current location as well as live location of the subject. The fuse & analyze module 110E receives the processed information from the module 110A-110D as input and further processes the information to sense whether the subject is under the influence of the alcohol. The memory 102 further includes a plurality of modules (not shown here) comprises programs or coded instructions that supplement applications or functions performed by the system 100 for executing different steps involved in the process sensing alcohol intoxication of the subject. The plurality of modules, amongst other things, can include routines, programs, objects, components, and data structures, which perform particular tasks or implement particular abstract data types. The plurality of modules may also be used as, signal processor(s), node machine(s), logic circuitries, and/or any other device or component that manipulates signals based on operational instructions. Further, the plurality of modules can be used by hardware, by computer-readable instructions executed by the one or more hardware processors 104, or by a combination thereof. The plurality of modules can include various sub-modules (not shown).

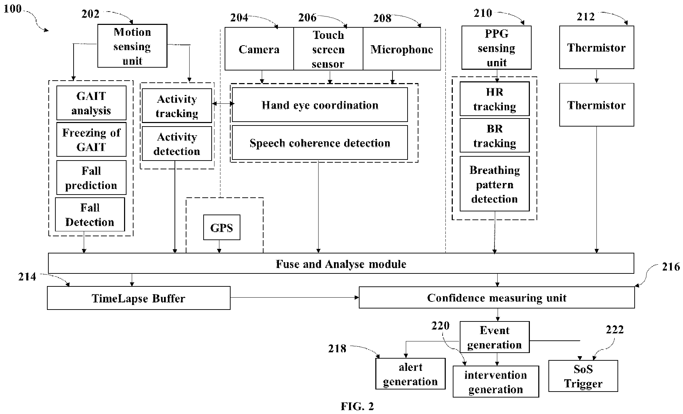

FIG. 2 illustrates in-built components of the wearable device 112 and the mobile device 114 and their contribution in analysing specific parameters vital to sense alcohol intoxication, according to some embodiments of the present disclosure.

As illustrated in FIG. 2, the system 100 for sensing the alcohol intoxication comprises of a plurality of modules which are functionally connected to the wearable device 112, the mobile device 114 and the cloud server 116 for receiving and processing the information and signals relevant for sensing the alcohol intoxication. The system 100 processes the alcohol sensing in two phases: Phase-I sensing and Phase-II sensing. The Phase-I sensing ascertains that the subject is under the influence of alcohol, and the Phase II sensing investigates if the subject needs assistance. Since, not all casual drinking episodes would need assistance, system should not send alerts, SoS to emergency contacts just for merely detecting intoxication. However, there might be episodes of over-consumption, where the subject may lose control and need help, and this is when the system should alert the emergency contacts and call for help immediately. Hence, the system treats these two separately.

**Phase-I sensing:** The system 100 utilizes a motion sensing unit 202 (an Inertial Motion Unit (IMU) sensors) present on the wearable device 112 and the mobile device 114. The motion sensing unit 202 includes accelerometer, gyroscope, and barometer. The accelerometer performs step counting, activity monitoring, or motion artwork and suppression. The gyroscope enables recording and analyzing parameters in real-time and in a non-invasive way. The barometer tracks the changes in elevation while performing physical activity such as climbing or hiking. The motion sensing unit 202 gathers raw data from the accelerometer, gyroscope, and barometer. The raw data collected is processed using a gait detection algorithm for gait analysis and freezing of gait detection. The resulting gait pattern represents subject's manner of walking. The human gait is defined as bipedal, biphasic forward propulsion of the center of gravity of the human body, in which there are alternate sinuous movements of different segments of the body with least expenditure of energy. Gait patterns may be unique to each individual and while under the influence of alcohol subject's may not be able to walk straight, and hence may produce abnormal gait pattern. Freezing of gait is characterized by brief episodes of inability to step or by extremely short steps that typically occur on initiating gait or on turning while walking. Based on recurring data received from motion sensing unit 202 trains the gait detection algorithm in such a way that if the gait pattern is slightly changed or the subject's center of gravity is changed due to any reason, the algorithm is fairly able to sense the abnormality. A time lapse buffer 206 performs gait activity recording and saves the data in cloud. The time lapse buffer 206 records multiple occurrence of specific events like improper gait, drinking gestures, etc. Such repeat occurrences in successions are used to evaluate overall magnitude of the event. Magnitude may differ from one sensing to another sensing. For example: in case of gait and drinking gesture it could be occurrence count, along with time distance between two occurrences; in case of fall detection magnitude may be force with which one falls. Thus, a location stamp data is created, and it gets recorded in the cloud database every time the algorithm processes the raw data. This can be utilized by the system 100 to analyze gait pattern variability in the absence and under the influence of the alcohol. The raw data gathered by the motion sensing unit 202 is further utilized for fall prediction and fall detection. Imbalance in gait by measuring parameters such as stance phase and balance leads to fall prediction and detection events. These events, if occur are used to trigger alarms and SoS respectively for facilitating immediate help by the subject. The activity tracking and activity detection basically keeps analysing raw data to identify all the activities detected through motion sensing unit and the analysis is then transferred to the cloud using time lapse buffer 206. Gait alone may not be used as a sole indicator for intoxication since gait patterns may vary even due to temporary injuries or pain or other medical conditions. This could lead to false alarms. Hence, to increase the confidence of prediction, other measures are also considered. Apart from gait , the raw data from motion sensing unit 202 detects hand-eye coordination and speech coherence detection. Activities such as eating, drinking, and smoking that involve hand gestures made while eating, drinking, and smoking are detected and classified respectively. The system 100 further utilizes camera 204, touchscreen sensor 206 and microphone 208 present on the wearable device 112 and the mobile device 114. The camera 204, touchscreen sensor 206 and microphone 208 captures audio data, video data, touch-based data that is further processed by the plurality of modules of the system 100. The system 100 further utilizes Photoplethysmograph (PPG) sensing unit present on the wearable device 112. The continuous monitoring of clinically relevant information such as hear rate and breathing pattern aims at the prevention, treatment, and management of diseases and the well-being of the users. The PPG sensor on wearable device 112 is used to detect abnormal heart rate, breathing rate, and abnormal breathing pattern. These markers are used to identify abnormality with confidence. Elevated heart rate "bEHr", dropping heart rate "bDHr", breathing rate "bBr" and breathing pattern "bBp" are some markers that are used. The system 100 further utilizes thermistor present on the wearable device 112. The thermistor provides body temperature data to the plurality of modules of the system 100. Further, system 100 provides location detection of the subject through Global Positioning System (GPS). The GPS data is processed through a plurality of modules to generate location field vector. The location is used to identify any publicly known location serving alcohol, and subject being present at such a location serves as a marker. Location is also used as a longitudinal marker, which over a period of time co-relates the gait analysis, hand gesture related data and other physiological abnormality along with subject's current location. The location stamp data may then suggest the probability of subject consuming alcohol while being at a specific geographic location. The inputs collected from motion sensing unit 202, camera 204, touch sense screen 206, microphone 208, PPG sensing unit 210 and thermistor 212 are processed through the plurality of modules of the system 100 to obtain various inferences about subject and the possibility of alcohol intoxication. The various inference so obtained are collated by fuse and analyze module 110E to determine whether the subject has consumed an alcohol and if the subject needed the SoS support. Once the fuse and analyze module 110E validates the intoxication of the subject, a confidence measuring unit 216 prompts the subject about the state of intoxication. The confidence measuring unit 216 generates a plurality of events like alert generation 218, intervention generation 220 and SoS trigger 222. The alert generation 218 attempts to warn the subject about the quantity of alcohol consumed, time duration of consumption and physiological abnormality detected by way of notifying the subject. The notification can be triggered through the wearable device 112 or the mobile device 114. The notification can be in the form of nudges alerting the subject; an audio warning the subject, a vibration in the specific pattern, a ring tone, a particular image, or an animation being displayed on the wearable device or the mobile device or the both. The intervention generation 220 attempts to try to make the subject aware about its state of intoxication. Also, the intervention generation 220 attempts to establish soft connect with the contact(s) specified by the subject as help/ assistance. The contact(s) specified by the subject to be reached out to support the subject in the state of intoxication. The soft connect can be differentiated with SoS to be the case wherein the help contacts may be notified about the state of the intoxicated subject. The intervention generation 220 attempts to check the attentiveness of the subject by launching a mobile device application on the screen of the mobile device. The mobile device application is an interactive application designed as game/ quizlet that precedes the lock screen of the mobile device. Based on the score of the mobile device application, the intervention generation 220 notifies the subject by multitude of ways mentioned above. The confidence measuring unit at 216 directly generates the SoS trigger in case fuse and analyzes module 110E to indicate any physiological abnormality to be attended immediately by the contact specified by the subject for help.

**Phase-II sensing:** The Phase-II sensing of the system 100 is used to detect if the subject is unable to take care of themself. The Phase II sensing is activated only after intoxication is detected. The confidence measuring unit 216 detects the confidence value crossing a set threshold. The system 100 uses the mobile device application to interact with the subject, to perform this analysis. This mobile device application precedes phone lock screen. It is launched when subject picks up the phone for use (after being detected for intoxication) the application will be shown to the subject. This application will require the subject to interact with it before they can use the phone. However, the application will not restrict the subject from using the phone. The application may randomly choose different methods to evaluate subject's attentiveness by asking them to perform one or more tasks. The attentiveness module executes phase-II sensing wherein it performs a check as to how responsive the user is for any kind of stimulus. This is a reflexive ability and cognitive ability checking measure wherein brain functioning of the subject in normal state and in the intoxicated state is compared. Once the subject performs the tasks irrespective of them doing it successfully or not, the application will let the subject proceed to use the phone normally. The system 100 uses motion sensing unit 202 for motion and activity tracking, camera 204 for gaze tracking, microphone 208 for voice and speech analysis to evaluate the subject's interaction with the devices during phase-II sensing as well. The system 100 will measure the quality of subject's hand-eye coordination, speech coherence, slurry ness in speech etc. This eventually lets the system decide if the subject is in control of mental faculties or SoS is advertent.

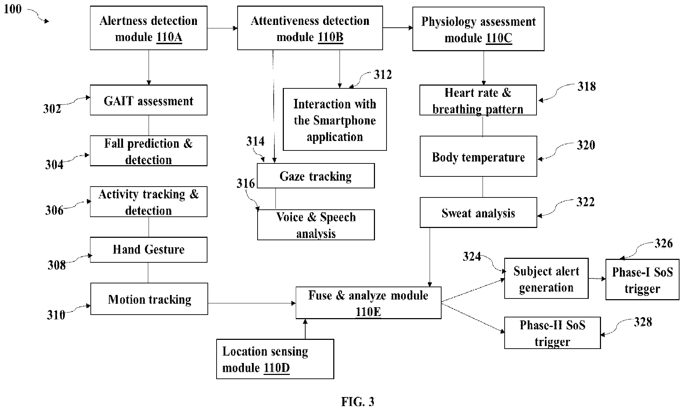

FIG. 3 illustrates a functional block diagram of an intoxication detection model comprising a plurality of modules sensing variation of physiological and physical parameters under the influence of the alcohol.

As illustrated in FIG. 3, the system 100 utilizes a plurality of components of the wearable device 112 and the mobile device 114. The plurality of components includes motion sensing unit (IMU), camera, microphone, touch screen sensor, PPG sensing unit, and thermistor. The plurality of components collects the raw data and process the collected data through alertness detection module 110A, attentiveness detection module 110B and physiology assessment module 110C. The alertness detection module 110A processes the raw data collected from the plurality of components of the wearable device 112 and the mobile device 114 to perform gait assessment 302, fall prediction & detection 304, activity tracking & detection 306, hand gesture 308 and motion tracking 310. The mobile device 114 utilizes the raw data collected over time to analyze gait pattern of the subject and trains the gait detection algorithm in such a way that if the gait pattern is slightly changed or the person's center of gravity is changed due to any reason, the algorithm is fairly able to sense the abnormality. Any abnormality in the gait 302 due to influence of the alcohol is calculated using below equation 1:

$$vGt = bGt.mGt.wGt \qquad (1)$$

where, vGt = bGt.mGt.wGt (1) Where "vGt" is a single vector indicating gait 306 measure, it is computed as product of "bGt" indicating gait abnormality, "mGt" is magnitude of abnormality and "wGt" is the weightage assigned to gait sensing in the overall computation. Magnitude of abnormality "mGt" is computed based on the number of abnormal gait events detected in a specific interval of time. Thereby increasing the confidence for the impending risk. The raw data gathered by the motion sensing unit 202 is further utilized for fall prediction and detection 304. Imbalance in gait by measuring parameters such as stance phase and balance leads to fall prediction and detection events. These events, if occur are used to trigger alarms and SoS respectively for facilitating immediate help by the subject. Therefore, fall prediction and detection is utilized in both phase-I and phase-II analysis to manage emergency situation. Further raw data from IMU is processed by the alertness detection module 110A for activity tracking and detection 306. The activity tracking and detection 306 analyzes the raw data to identify all the activities detected through motion sensing unit. The activities analyzed include walking, standing, body movements etc. Further, sensing of the alcohol intoxication through hand gesture 308 involves plurality of activities to be analyzed that are associated with hand movement such as eating, drinking, or smoking. The hand gestures 308 made while eating, drinking, and smoking are detected and classified using below equation 2:

$$vAct = \frac{\sum_{i=0}^{n-1} bAct_i. mAct_i. wAct_i}{n} \tag{2}$$

where, a single vector indicating confidence using activities is represented by "vAct"; drinking gesture (detected by analysing hand movements) - "bAct1" are flags and each activity carries a magnitude "mAct1" and a weightage in overall computation "wAct1"; smoking gesture (detected by analysing hand movements) - "bAct2", "mAct2" & "wAct2"; detection of deterioration in hand movement while drinking - "bAct3", "mAct3" & "wAct3"; detection hand-eye coordination "bAct4", "mAct4" & "wAct4"; detection of sudden erratic hand movements as "bAct5", "mAct5" & "wAct5".

Similarly, any further activities involving hand movement would be added to obtain an indicator for the hand gesture 308. Further, the alertness detection module 110A processes the data received from accelerometer present on the mobile device 114 for motion tracking 310. The motion tracking 310 measures and analyzes acceleration, side to side, up and down, and forward and backward movements while walking, and predicts whether the subject is under the influence of the alcohol. As the alertness detection module 110A processes the raw input data and senses that the subject is under the influence of the alcohol; the system 100 triggers the attentiveness detection module 110B. This trigger may be a confirmation of the findings of the alertness detection module 110A. The attentiveness detection module 110B senses a responsiveness to stimuli of the intoxicated subject. The responsiveness to stimuli basically checks the reflexive ability and comprehensive ability of the subject under the influence of the alcohol. The reflexive ability deteriorates under the influence of the alcohol. E.g. if a ball is thrown pointing towards the face of the subject, his normal reflex will be to move his face away from the direction of the ball to duck away from the ball or catch the ball. However, under the influence of alcohol, the subject may not be able to act in accordance. This could be of a concern. Similarly, comprehensive ability deteriorates under the influence of the alcohol. E.g. If person is not able to comprehend, they may be gullible and can be easily conned into doing something that they may not otherwise do when they are in their conscious mind. Therefore, subject's responsiveness to stimuli is calculated by aggregating the score of the reflexive ability and the comprehensive ability. The attentiveness detection module 110B senses the subject's responsiveness to stimuli under the influence of alcohol by analyzing the interaction of the subject with the mobile device possessed by the subject. The attentiveness detection module 110B launches the mobile device application 312 on the screen of the subject's mobile device. The mobile device application 312 is a privileged application that precedes the lock screen of the mobile device. It is designed as an interactive application based on short game/ quizlet to sense attentiveness of the subject based on his interaction with the application 312. The application 312 requires the subject to interact with it before the subject can use his mobile device. In an embodiment, the application 312 randomly chooses different methods to evaluate subject's alertness by asking the subject to perform some task. For example: the application 312 may ask the subject to:

- draw a shape on phone screen,
- draw a shape in air (using the arm in which subject is holding the phone),
- look at the phone screen while moving the hand holding the phone without turning the head,
- look at moving objects on the phone screen,
- say a phrase shown on the screen
- read the given paragraph and choose the appropriate summary from the options below, etc.

The above tasks are designed to check either reflexive ability or the comprehensive ability of the subject under the influence of the alcohol. The Subject may be asked to do perform one or more tasks.

Once the subject performs these tasks irrespective of them doing it successfully or not, the application 312 allows the subject to use the mobile device normally. The attentiveness detection module 110B processes the response of the subject to obtain a score for the responsiveness to stimuli which is derived by aggregating a score for reflexive ability and a score for comprehensive ability as equation 3:

$$vResponsivenessToStimuli\ (R_{sti}) = \frac{vComprehension + vReflex}{2} \tag{3}$$

Further, the attentiveness detection module 110B performs gaze tracking 314. The gaze tracking 314 involves analyzing the eye movements of the subject. The eye movement changes drastically when the subject is under the influence of the alcohol. According to an embodiment, gaze tracking 314 is performed through various computer implemented processes like perception, attentional bias, memory, executive functions, prevention message processing etc. The gaze tracking data indicates a visuo-motor impairment (related to reduced cerebellar functioning) following alcohol intoxication, together with reduced memory and inhibitory control of eye movements. Further, the attentiveness detection module 110B performs voice and speech analysis 316 by processing the voice signals received from the microphone of the mobile device. Because the motor speech and voice act represent the output of several high-level, integrated systems (sensory, cognitive, and motor), it is reasonable to suggest that speech too may be susceptible to the degrading effects of alcohol consumption. Alcohol is generally considered to be a central nervous system depressant. According to an embodiment, the voice and speech analysis 316 involves identifying impaired intellectual functioning, reaction time, coordination, reflexes, and nerve transmission. Alcohol consumption is also thought to produce changes in speech production that are often described as "slurred speech" which is sensed by the attentiveness detection module 110B. As the attentiveness detection module 110B processes the response of the subject based on his interaction with the mobile device application, and senses that the subject is under the influence of the alcohol; the system 100 triggers the physiology assessment module 110C. This trigger is a kind of re-confirmation of the findings of the alertness detection module 110A and attentiveness detection module 110B for initiating phase-I and phase-II sensing respectively. The system 100 comprises of the physiology assessment module 110C. The processing of the physiology assessment module 110C involves active as well as passive monitoring. Therefore, output of the physiology assessment module 110C contributes both in phase-I as well as phase-II sensing. The physiology assessment module 110C monitors heart rate and breathing pattern 318, body temperature 320 and performs sweat analysis 322. The physiology assessment module 110C utilizes PPG sensors to obtain heart rate 318 of the subject wearing wearable device 112 and possessing the mobile device 114. Elevated heart rate" bEHr", dropping heart rate" bDHr", breathing rate "bBr" and breathing pattern "bBp" are some markers that are used. In an embodiment, body temperature may also be obtained from the wearable device. When available, the change in body temperature 320 "bTmp" can also be used as a signature. With "bPpm[]" being an array holding all the physiological indicators, "mPpm" being an array of magnitudes of all markers, and "wPpm" being weights for each marker in overall computation. A single vector indicating physiological abnormality is obtained as "vPpm" below as equation 4:

$$vPpm = \frac{\sum_{i=0}^{n-1} bPpm_i . mPpm_i . wPpm_i}{n}$$

$$(4)$$

Further, the physiology assessment module 110C utilizes wearable device 112 to receive inputs for sweat analysis 322. According to an embodiment, the sample for sweat analysis 322 can be obtained through a non-invasive microfluidic sensing patch that measures the ethanol levels in sweat from the subject's arm. According to another embodiment, the electrochemical biosensors in the wearable sensing platforms are utilized. The system 100 further sense the location of the subject by a location sensing module 110D. The location sensing module 110D receives the GPS location. Further, the location sensing module 110D establishes the context of GPS location of the subject with that of the availability of the alcohol. The location thus identified could be a publicly known location serving alcohol (e.g. restaurants, bars etc.) or the location friend's place where the subject is known to consume alcohol based on his past record/ history. Any such correlation found by the location sensing module 110D is used as a longitudinal marker, which over a period of time co-relates the vGt, vAct, vPpm along with subject's current location. The location stamp data may then suggest the probability of subject consuming alcohol while being at a specific geographic location and is given by an equation 5:

$$vLoc = \frac{\sum_{i=0}^{n-1} bLoc_i . mLoc_i . wLoc_i}{n}$$

$$(5)$$

where "vLoc" is one single location vector indicating confidence for alcohol consumption. Finally, the individual outcome of alertness detection module 110A, attentiveness detection module 110B, physiology assessment module 110C and location sensing module 110D is input to the fuse and analyze module 110E and the fuse and analyze module 110E process the responses of all the modules and sense whether subject is intoxicated with the alcohol and whether the subject is to be warned or an SoS is to be raised for the contacts specified by the subject for help/ assistance in case of alcohol intoxication. The fuse and analyze module 110E give the intoxication confidence score as equation 6:

$$vIntoxication = \left(\frac{vGt + vPpm + vAct + vLoc}{4}\right) * R_{sti}^{-1}$$

$$(6)$$

where, the inverse of $R_{sti}$ is employed to adjust the overall intoxication confidence score. As $R_{sti}$ increases, it indicates

higher user attentiveness, while a lower $R_{sti}$ suggests greater user vulnerability.

Based on the intoxication confidence score, the system 100 triggers alert generation 320 to the subject by the multitude of ways wherein the subject is made aware that he is intoxicated with the alcohol. When the intoxication confidence score crosses the set threshold, the system 100 triggers phase-I SoS 326 or phase-II SoS 328. The phase-I SoS 326 is triggered when the fuse and analyze module 110E sense an abnormality associated with the physiological parameters like abnormal heart rate, trip or fall and the like.

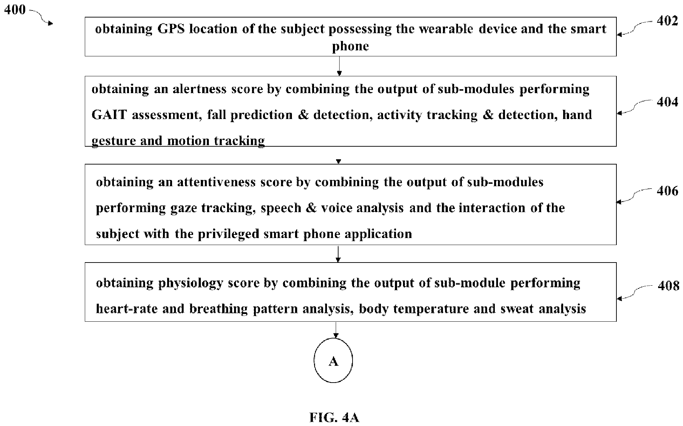

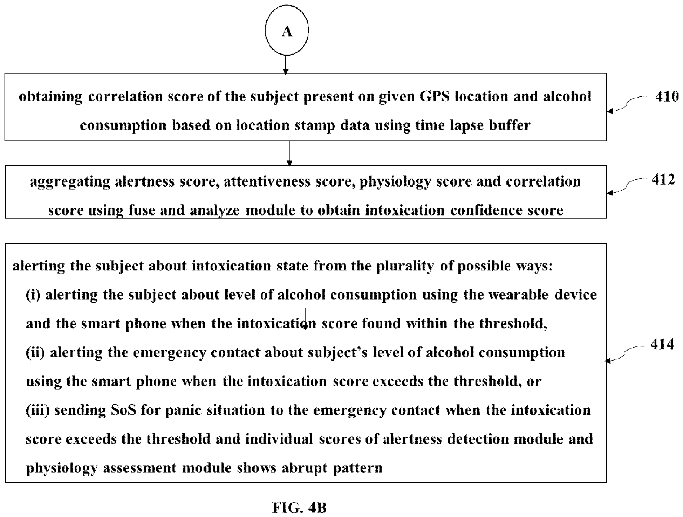

FIGS. 4A and 4B are flow diagrams of an illustrative method 400 for detection of alcohol intoxication in the subject possessing the wearable device 112 and carrying the mobile device 114, according to some embodiments of the present disclosure.

The steps of the method 400 of the present disclosure will now be explained with reference to the components or blocks of the system 100 as depicted in FIG. 1 through FIG. 10. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously. At step 402 of the method 400, the one or more hardware processors 104 of the cloud server 116 are configured to obtain GPS location of the subject possessing the wearable device and the mobile device. The GPS enabled wearable device such as smart watch and the mobile device such as smartphone not only sense the current location of the subject but also keeps a record of the locations visited by the subject . In an embodiment, the intoxication detection model 110 receives GPS location from mobile device. All the location record (as location stamp data) is utilized by the intoxication detection model 110 to sense alcohol detection of the subject. At the step 404 of the method 400, the one or more hardware processors 104 are configured to obtaining an alertness score of a subject by combining the output of motion sensing unit (e.g. IMUs), gait detection module, activity tracking and detection module. The alertness detection module 110A acquires motion sensing data of the subject comprising gait pattern, activity sensing and hand gestures using inertial motion sensors of the mobile device and a wearable device worn by the subject, wherein the motion sensing data is processed to obtain an alertness score. The alertness detection module 110A receives input from the IMU sensors and other activity and motion detection applications present in the wearable device 112 and the mobile device 114. The alertness detection module 110A processes the input through a plurality of sub-modules to obtain motion sensing data. The plurality of sub-modules performs alertness sensing activities through gait assessment, activity tracking and detection, motion tracing and detection and hand gesture analysis. Further, the alertness detection module 110A utilizes the raw data collected over time to analyze human gait pattern of the subject and trains the gait detection algorithm in such a way that if the gait pattern is slightly changed or the person's center of gravity is changed due to any reason, the algorithm is fairly able to sense the abnormality. Further, the alertness detection module 110A senses the alcohol intoxication through hand gesture through the activity detection sub-module. The activity detection sub-module analyzes the plurality of activities that are associated with hand movement such as eating, drinking, or smoking. Further, the alertness detection module 110A processes the data received from accelerometer present on the mobile device for motion tracking. The motion tracking measures and analyzes acceleration, side to side, up and down, and forward and backward movements while walking, and predicts whether the subject is under the influence of the alcohol. At step 406 of the method 400, the one or more hardware processors 104 are configured to obtain an attentiveness score of the subject by combining the output of sub-modules performing gaze tracking, speech & voice analysis, and the interaction of the subject with the privileged mobile device application. The attentiveness detection module 110B acquires a cognitive ability sensing data of the subject from the mobile device based on a response of the subject to a plurality of activities performed on the mobile device, wherein the plurality of activities comprising (i) interacting with an application installed on the mobile device, personalized for the subject, (ii) performing gaze tracking by utilizing a camera of the mobile device, (iii) performing voice and speech analysis by utilizing a microphone of the mobile device, wherein the cognitive ability sensing data is processed to obtain an attentiveness score. The attentiveness detection module 110B senses the alcohol intoxication of the subject by analyzing the interaction of the subject with the mobile device possessed by the subject. The attentiveness detection module 110B launches the mobile device application on the screen of the subject's mobile device. The mobile device application is a privileged application that precedes the lock screen of the mobile device. It is designed as an interactive application based on short game/ quizlet to sense attentiveness of the subject based on his interaction with the application. The application requires the subject to interact with it before the subject can use his mobile device. In an embodiment, the application randomly chooses different methods to evaluate subject's attentiveness by asking the subject to perform some activity/task. Once the subject performs these tasks irrespective of them doing it successfully or not, the application allows the subject to use the mobile device normally. The attentiveness detection module 110B processes the response of the subject based on its interaction with the application. Further, the attentiveness detection module 110B performs gaze tracking analysis and voice and speech analysis. The gaze tracking involves analyzing the eye movements of the subject. The eye movement changes drastically when the subject is under the influence of the alcohol. Further, the voice and speech analysis sub-module process the voice signals received from microphone of the mobile device. Because the motor speech and voice act represent the output of several high-level, integrated systems (sensory, cognitive, and motor), it is reasonable to suggest that speech too may be susceptible to the degrading effects of alcohol consumption. By combining the output of cognitive ability sensing data involving interaction with the application, gaze tracking and voice & speech analysis, the attentiveness score is derived. At step 408 of the method 400, the one or more hardware processors 104 are configured to obtain a physiology score by combining the output of sub-module performing heart-rate and breathing pattern analysis, body temperature and sweat analysis. The physiology assessment module 110C acquires physiology sensing data from a plurality of sensors on the wearable device and the mobile device, the physiological sensing data comprising (i) body temperature via thermistors, (ii) sweat analysis based on sweat sensing via transdermal sensors, (iii) heart rate variability via Photoplethysmograph (PPG) sensors, wherein the physiology sensing data is processed to obtain a physiology score. The physiology assessment module 110C utilizes PPG sensors to obtain heart rate of the subject. Elevated heart rate, dropping heart rate, breathing rate, and breathing pattern are some markers processed by the physiology assessment module 110C based on input received from PPG sensors. Next, the physiology assessment module 110C processes the input received from the transdermal sensors present on the wearable device to perform sweat analysis. Next, the physiology assessment module 110C processes the input received from the thermistor to sense body temperature at the time of performing physiology scoring. At the step 410 of the method 400, the one or more hardware processors 104 are configured to receive a correlation score of the subject present on given GPS location and alcohol consumption based on the location stamp database using the location sensing module 110D. The location sensing module 110D utilizes a time lapse buffer. Once, the subject agrees to track GPS location while personalizing the mobile device application during on-boarding, the time lapse buffer keeps a track of all the GPS locations visited by the subject and correlates the episodes of alcohol consumption. The location sensing module 110D then derives a correlation score based on specific GPS locations and the episodes of alcohol consumption. At the step 412 of the method 400, the one or more hardware processors 104 are configured to aggregate the alertness score, the attentiveness score, the physiology score, and the correlation score (based on GPS location) using fuse and analyze module 110E to obtain the intoxication confidence score. The individual outcome of alertness detection module 110A, attentiveness detection module 110B, physiology assessment module 110C and location sensing module 110D is input to the fuse and analyze module 110E and the fuse and analyze module 110E process the responses of all the modules and sense whether subject is intoxicated with the alcohol and whether the subject is to be warned or an SoS is to be raised for the contacts specified by the subject for help/ assistance in case of alcohol intoxication. The fuse and analyze module 110E give the intoxication confidence score. A threshold value is pre-defined in the system 100 based on tolerance of sensitiveness of physiological parameters as well as subjects own personalized information given at the on-boarding. In an embodiment of the present invention, when a threshold for the intoxication confidence score is >t1%, an alert is sent to the user. When the threshold for the intoxication confidence score is >t2%, the alert is sent to the user as well as to the emergency contact. And, when the intoxication confidence score is >t3%, then the SoS is sent to the emergency contact. The intoxication detection model 110 is trained to identify the threshold limits for each module as tolerance limits and the threshold limit varies from one individual to another individual based on adverse impact of the alcohol on the subject. At the step 414 of the method 400, the one or more hardware processors 104 are configured to alert the subject about level of alcohol consumption through plurality of possible ways using the wearable device 112 and the mobile device 114 when the intoxication confidence score found within the threshold. The plurality of ways involves alerting/ warning the subject through notification or popping-up some nudges or triggering some recorded message or a warning tone on the screen of the wearable device. Similarly, alerting/ warning the subject through notification or popping-up some nudges or triggering some recorded message or a warning tone on the screen of the mobile device. At the step 416 of the method 400, the one or more hardware processors 104 are configured to alert the emergency contact about subject's level of alcohol consumption by alerting the emergency contact using the mobile device when the intoxication confidence score exceeds the threshold. The emergency contact specified by the subject during on-boarding is notified as the intoxication confidence score exceeds the threshold sensing that the subject may need help/ assistance. At the step 418 of the method 400, the one or more hardware processors 104 are configured to send SoS for panic situation to the emergency contact when the intoxication confidence score exceeds the threshold and individual scores of alertness detection module and cognitive scoring module shows abrupt pattern. This is an emergency triggered fallback arrangement of the system 100 wherein the subject is considered to be in the state of immediate assistance may be due to abnormal physiological parameters (such as sudden heart rate change, acute breathing difficulty etc.) or abnormality detected in the motion (e.g. sudden slip-trip-fall situation).

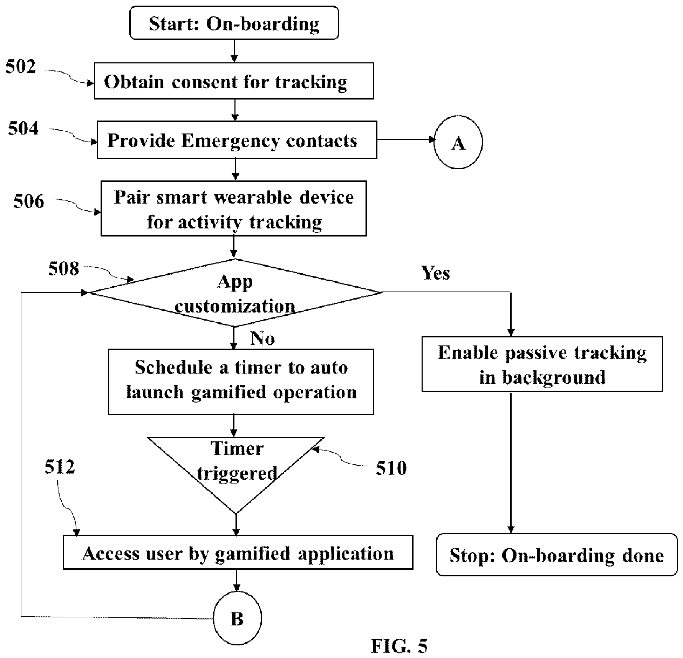

FIG. 5 is a flow diagram for an on-boarding the mobile device application, according to some embodiments of the present disclosure.

As illustrated in FIG. 5, the system 100 executes the detection of alcohol intoxication based on the information available in the onboarding application. The system 100 performs alcohol intoxication detection of a person by using various components present in the wearable device 112 and the mobile device 114. The system 100 to function in the appropriate manner, the subject has to possess both the wearable device 112 and the mobile device 114. As a pre-requisite, on-boarding step is to be executed by the subject for the performance of the system 100. The onboarding application involves (a) declaration of subject's identity, (b) declaration of subject's emergency contacts, (c) subject's consent for passive tracking of activities associated with functioning of the detection of alcohol intoxication, and (d) training of the plurality of models of the system 100 based on raw data generated through the plurality of the components of the wearable device 112 and the mobile device 114. Training of the intoxication detection model 110 is an essential step for more accurate sensing of alcohol intoxication in the subject. Once the subject agrees for monitoring of body parameters, the model keeps analysing the body parameters passively and trains itself for the better performance. To explain the step-by-step process of on-boarding, the subject in possession of wearable device 112 and the mobile device 114 is referred to as "user". At the step 502, the onboarding application seeks the users consent for tracking of various parameters to be utilized by the model for sensing alcohol intoxication of the subject. At the step 504, the application prompts the user to specify the emergency contact details. As soon as the user specifies the emergency contact, a communication goes to the emergency contact about specifying him/ her as the emergency contact as well as a consent is sought from the emergency contact. This task of sending a verification communication (e.g. a verification link) and receiving the confirmation is specified as the task "A". At the step 506, the application prompts the user to pair the associated wearable device with the mobile device. At the step 508, the user has to perform application customization. In the customization the system tries to understand the normal activities and normal pattern of the user, for example: normal GAIT, walking, running, climbing, eating, drinking patterns, breathing patterns, etc. Through an interface (e.g.) user is asked to provide a plurality of questions or information relevant for functioning of the plurality of modules of the intoxication detection model. After successful customization, the onboarding application starts tracking the various parameters vital for the intoxication detection of the subject. If the user is not able to customize the application may be due to a plurality of reasons like unavailability of the health-related information at the time of application installation or connectivity challenge or time constraint; then the application prompts the user to schedule a timer to auto launch the application along with gamified operation wherein the user can complete the customization at later time And, the application can record normal response. The is internal to the system. It is similar to the interactive mode. It will popup preceding lock screen when user pickups the mobile device. And there is time delay between each interactive mode, as described below in subsequent comments.

Meanwhile, the application continues to run in the background. At the step 510, the time triggers based on specified time and prompts the user to complete the customization. At the step 512, the application launches automatically and prompts the user to customize the application. This launch is by way of preceding lock screen whenever user wakes the phone from idle state. Based on user interaction, the application records the responses of the user and this way, on-boarding step gets completed. If user did not complete the customization, the application takes the user to step 508 to complete the customization at a later time. This is shown as task "B" in the flow diagram.

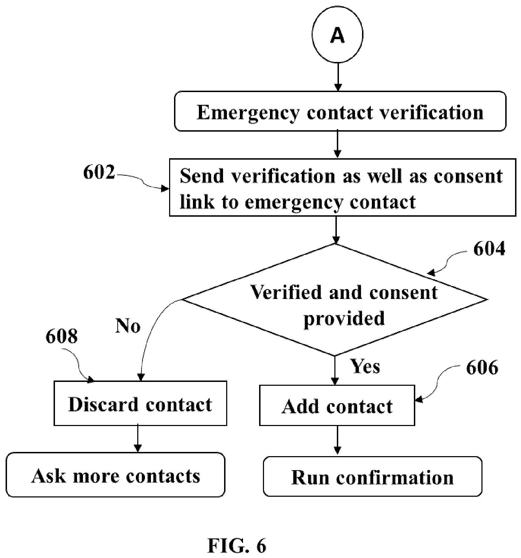

FIG. 6 is a flow diagram of indicating steps for emergency contact verification performed by the method 400, according to some embodiments of the present disclosure.

As illustrated in FIG. 6, emergency contacts specified by the user are verified by sending a communication to the specified contacts. At the step 602, the onboarding application sends a verification link to the contact(s) specified by the user. At the step 604, if the contact receiving the verification link provides the consent, the contact gets added to the onboarding application as the SoS/ emergency contact. If the contact receiving the verification link does not provide the consent, the verification fails, and the application prompts the user to specify another contact.

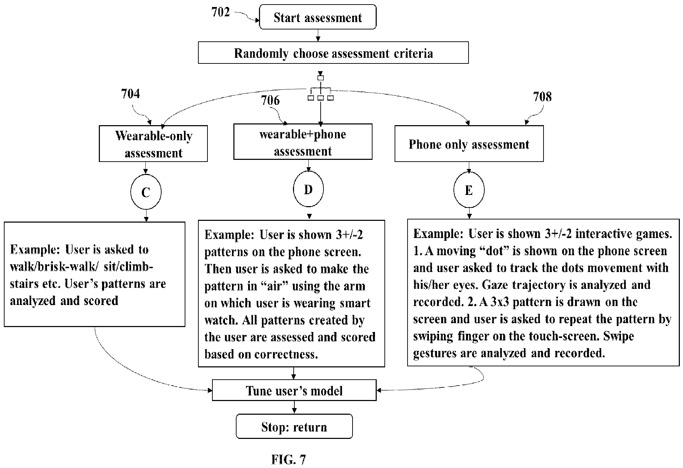

FIG. 7 illustrates mobile device application-based assessment of the subject under the influence of alcohol, according to some embodiments of the present disclosure.

As illustrated in FIG. 7, the mobile device application is utilized during the onboarding as well as the model training apart from its utilization in phase-II. The launch of the mobile device application during onboarding assessment involves the wearable device 112 or the mobile device 114 or both. Similarly, for training of the plurality of modules, the mobile device application involves the wearable device 112 or the mobile device 114 or both. At the step 702, various gamified tasks were presented randomly to the subject during the onboarding as well as the model training. The (C), (D) and (E) represent different tasks in Phase II sensing. At the step 704, the task "C" involves only the wearable device 112. The application performs assessment of the user using the wearable device. For E.g., the application asks the user to walk/ brisk-walk/sit/ climb-stairs/ etc. The user patterns are analyzed and scored. At the step 706, the task "D" involves both the wearable device 112 as well as the mobile device 114. The application presents the user with 3+/-2 patterns on the phone screen. Then user is asked to make the pattern in "air" using the arm on which user is having the wearable device 112. All patterns created by the user are assessed and scored based on their correctness. And average of all scores is recorded. At the step 708, the task "E" involves only the mobile device. The user is shown 3+/-2 interactive games. E.g. (1). A moving "dot" is shown on the phone screen and user asked to track the dots movements with his/ her eyes. Gaze trajectory is analyzed and recorded; (2). A 3x3 pattern is drawn on the screen and user are asked to repeat the pattern by swiping finger on the touch-screen. Swipe gestures are analyzed and recorded. These kind of task-based assessment tune the model for detecting alcohol intoxication in the person.

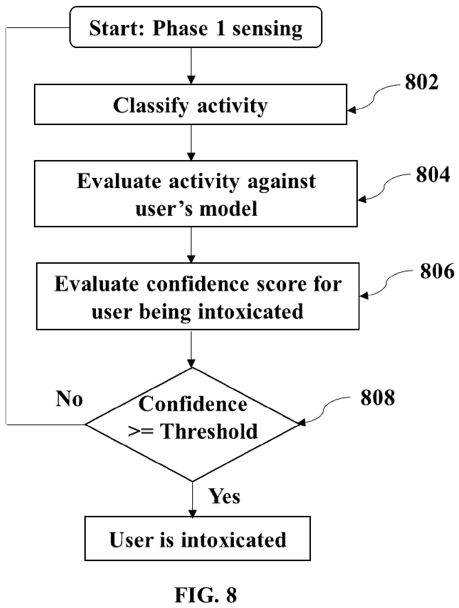

FIG. 8 is a flow diagram or phase-I sensing as performed by the method, according to some embodiments of the present disclosure.

As illustrated in the FIG. 8, the process that collaboratively uses a subject's wearable device 112 and the mobile device 114 to passively sense their intoxication level from the plurality of parameters being captured and processed though the trained model 110. The passive monitoring begins with Phase-I sensing. The subject's activities are tracked to ascertain if the subject is intoxicated. It is passive monitoring because system does not require subject to actively interact with it. It runs like a background service. All the vitals like gait pattern, heart rate, breathing pattern, sweat, body temperature, hand gesture, and motion are being monitored passively by the trained model 110 and outcomes are processed through respective modules (110A-110E). At the step 802, as the system 100 receives the activity input, it classifies the activity and sends it to the respective module for further processing. At the step 804, the processed activity is analyzed against the trained model 110 to ascertain unusual changes in the respective activities which can give a sense of the subject being under the influence of the alcohol. At the step 806, fuse and analyze module 110E receives outcome of the activity through the respective module and calculates the confidence score for the subject being intoxicated. At the step 808, the confidence score is scanned against the set threshold. If the confidence score found to exceed the set threshold, the subject is categorized as intoxicated. If the confidence score is found to be within the threshold, the system 100 continues to passively monitor the subject.

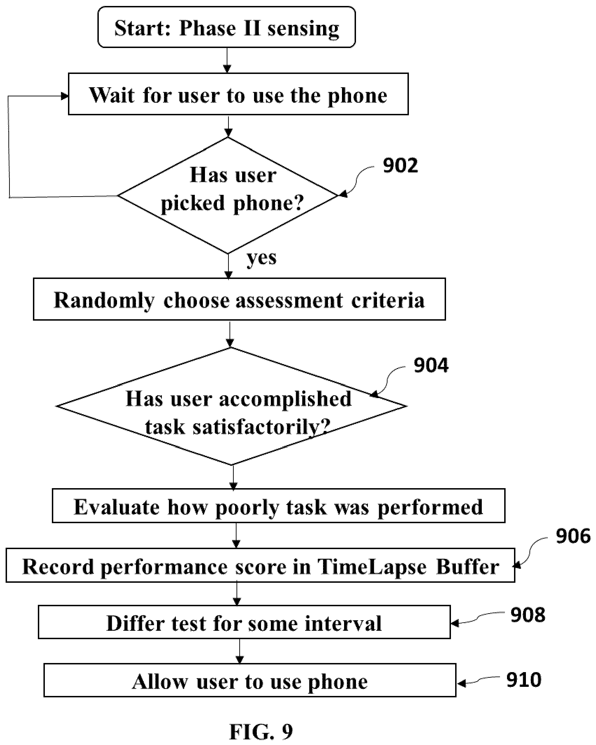

FIG. 9 is a flow diagram for phase-II sensing as performed by the method 400, according to some embodiments of the present disclosure.

As illustrated in the FIG. 9, the system 100 executes the Phase-II sensing. The Phase-II sensing initiates while the subject is detected as intoxicated through Phase-I scoring and picks up the mobile device for use. As the subject holds the mobile device, the mobile device application pops-up on the phone before the phone lock screen. The mobile device application tries to interact with the subject by way of game/quizlet interface wherein the subject is prompted to perform certain activity/ task. At the step 902, the system 100 sense that the subject has lifted the mobile device for the use. With this sense, the mobile device application pops up at the screen preceding the screen lock and tries to tries to interact with the subject by way of game/quizlet interface wherein the subject is prompted to perform certain activity/ task. At the step 904, based on the subject's interaction with the game/quizlet, the system 100 sense the subject has accomplished the tasks satisfactorily or not. This evaluation is based on the response of the subject to the activity/ task. At the step 906, once the subject is detected to have interacted with the activity, the score is calculated based on subject's response to the activity. At the step 908, the system controls popping-up gamified interaction preceding the lock screen. At a time, one game is presented, and the application sleeps until a specific time. This provision is to ascertain decent time gaps between the subsequent interactive episodes and not troubling the user in utilizing basic functionalities of the mobile device. At the step 910, if the user responds to the game/ quizlet, the score is recorded in the timelapse buffer. The record created in timelapse buffer is utilized in ascertaining the behavior and pattern of the subject and used by the model for on-the go training. The mobile device application lets the subject use the mobile device. Simultaneously, the score is scanned against the set threshold score and the further course of action is decided based on the score.

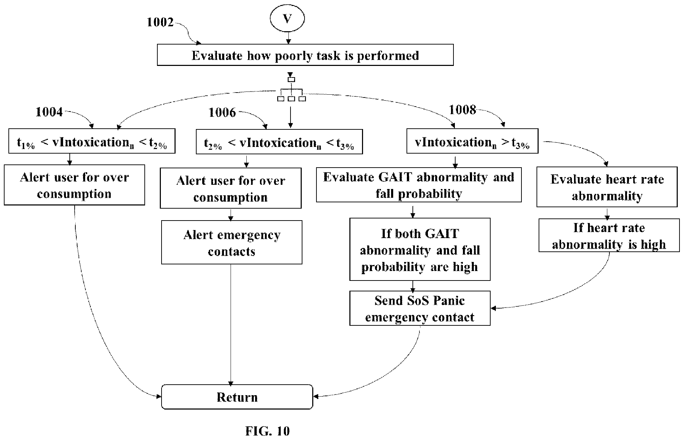

FIG. 10 illustrates a plurality of alert mechanism triggered by the intoxication detection model based on scoring mechanism, according to some embodiments of the present disclosure.

As illustrated in FIG. 10, the system 100 performs a plurality of operations based on the score obtained for the subjects' interaction with the mobile device application. Based on this evaluation, the system 100 executes a decision-making process followed by issuing alerts/intervention and SoS messages to the emergency contacts. The alert messages sent to emergency contact also include current location of the subject, while the SoS message include live location of the subject, until emergency contact cancels it. Therefore, the system assists the emergency contact to trace the user through the live location tracking.

At the step 1002, the system 100 calculates the score based on the responses of the subject against each activity/task assigned to him in the gamified application. Further, the score is normalized using below equation 7, to obtain the normalized score ($vIntoxication_n$). If the normalized score comes between $t_1\%$ and $t_2\%$ (i.e. $t_1\% < vIntoxication_n < t_2\%$), the system 100 warns the subject about over-consumption of the alcohol. If the score comes between $t_2\%$ and $t_3\%$ (i.e. $t_2\% < vIntoxication_n < t_3\%$) the system 100 warns the subject about over-consumption of the alcohol as well as sending the alert to the emergency contact about over-consumption of the alcohol by the subject. If the score comes $> t_3\%$ (i.e. $vIntoxication_n > t_3\%$), the system 100 further verifies the state of intoxication by analyzing gait abnormality at the current instance and calculates fall probability. Simultaneously, the system 100 obtains heart rate. If gait abnormality, fall probability and heart rate abnormality are found to be on a higher side, the system 100 classifies it as a panic situation and sends an SoS to the emergency contacts. The range for intoxication confidence vector *(vIntoxication)* is normalized to range 0 to 1 or 0% to 100% by calculating the minimum (min) and maximum (max) value of the *vIntoxication.* The minimum value is defined by considering the subject'/user's normal behavior and parameters received from the plurality of modules when the user is not intoxicated. The minimum value is obtained and calculated during onboarding process where the user is considered to be in normal senses and not under any intoxication. The maximum value is defined by considering user's worst abnormal behavior and parameters received from the plurality of sensing modules when the user is heavily intoxicated. In an embodiment, the maximum value is calculated by considering the worst-case scenario or can also be determined by pre-training the model with generic available data of intoxicated subjects from the literature. Using these min and max values, normalization is performed on the scale of 0 to 1 or 0% to 100% (these become new_min and new_max). The formula for normalization to obtain normalized intoxication confidence score (*vIntoxication_norm*) of the intoxication confidence score calculated in equation (6) is represented by the equation 7:

$$vIntoxication_n = \left(\frac{(vIntoxication - min)}{(max - \min)} * (new\_max - new\_min)\right) + new\_min \qquad (7)$$

for example, if new_min = 0 and new_max = 1, substituting the values in the equation (7), the normalized intoxication confidence score is represented as:

$$vIntoxication_n = \left(\frac{(vIntoxication - min)}{(max - \min)} * (1 - 0)\right) + 0 \qquad (8)$$

which is simplified by,

$$vIntoxication_n = \frac{(vIntoxication - min)}{(max - min)} \qquad (9)$$

This is the simple normalized *vIntoxication* vector in the scale of 0 to 1.

This normalization process is implemented to the final output of the vector (in this case vintoxication of equation (6)). Further, derived output of the Intoxication vector ($vIntoxication_n$) is utilized to trigger the alert as a result of Phase-I sensing or as Phase-II sensing. This range is further divided into three parameters (ti, $t_2$ and $t_3$ (defined earlier)) by taking percentages of min and max. For example, $t_1$ is 25% more of min value. If min is 10, then $t_1$ will be (10 + 25% of 10 ) = (10 + 2.5) = 12.5. Therefore, $t_1$ = 12.5. If the $vIntoxication_n$ is above 12.5, then an alert will be sent as a result of phase-II sensing. This is how system keeps sending alerts based on the normalized intoxication confidence score.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of invasive way of sensing alcohol intoxication by utilizing sensors and applications pre-installed in an IoT enabled wearable device and a mobile device. The cumulative scoring-based method precisely monitors physiological parameters, cognitive parameters, and physical parameters of the person under the influence of the alcohol. A score is assigned to each activity being monitored by the system and the cumulative score is obtained by adding the individual score of each activity. The cumulative score is matched against the threshold score to decide a plurality of possible ways of alerting the person under the influence of the alcohol or to the emergency contact of the person. With the alert mechanism, either person becomes conscious about the influence of the alcohol being already consumed, or the emergency contact become aware about the intoxication state of the person to arrange timely assistance.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

**1.**   A method for alcohol intoxication detection, the method comprising:

> receiving, via one or more hardware processors of a cloud server, a global positioning system (GPS) location of a subject from a mobile device carried by the subject;
> acquiring, via the one or more hardware processors, motion sensing data of the subject comprising gait pattern, activity sensing and hand gestures using inertial motion sensors of the mobile device and a wearable device worn by the subject;
> acquiring, via the one or more hardware processors, cognitive ability sensing data of the subject from the mobile device based on a response of the subject to a plurality of activities performed on the mobile device, wherein the plurality of activities comprising
>
>> (i) interacting with an application installed on the mobile device, personalized for the subject,
>> (ii) performing gaze tracking by utilizing a camera of the mobile device,
>> (iii) performing voice and speech analysis by utilizing a microphone of the mobile device, wherein the cognitive ability sensing data is processed to obtain an attentiveness score;
>
> acquiring, via the one or more hardware processors, physiology sensing data from a plurality of sensors on the wearable device and the mobile device, the physiological sensing data comprising (i) body temperature via thermistors, (ii) sweat analysis based on sweat sensing via transdermal sensors, (iii) heart rate variability via Photoplethysmograph (PPG) sensors, wherein the physiology sensing data is proceeed to obtain a physiology score;
> obtaining, via the one or more hardware processors, a correlation score using a location sensing module wherein the correlation score is derived by a time lapse buffer based on presence of the subject on a given GPS location and episodes of alcohol consumption;
> aggregating, via the one or more hardware processors, the alertness score, the attentiveness score, the physiology score, and the correlation score to obtain an intoxication confidence score of the person; and

alerting, via the one or more hardware processors, one of the subject and an emergency contact about the intoxication state of the person in accordance with the intoxication confidence score.

2. The method as claimed in claim 1, wherein the intoxication confidence score to sense the state of alcohol intoxication of the subject is calculated by an equation:

$$vIntoxication = \left(\frac{vGt + vPpm + vAct + vLoc}{4}\right) * R_{sti}^{-1}$$

wherein $vGt$ is an abnormality in the gait pattern, $vAct$ is an abnormality in the hand gesture, $vPpm$ is a physiological abnormality, $vLoc$ is a correlation score and $R_{sti}$ is an abnormality in responsiveness to stimuli.

3. The method as claimed in claim 2, wherein $vGt$, the abnormality in the gait pattern due to alcohol intoxication is calculated by an equation:

$$vGt = bGt.mGt.wGt$$

bGt indicates gait detection, mGt is a magnitude of abnormality associated with detected gait, and wGt is the weightage assigned to the detected gait in the overall computation.

4. The method as claimed in claim 2, wherein $vAct$, the abnormality in the hand gestures due to alcohol intoxication is calculated by an equation:

$$vAct = \frac{\sum_{i=0}^{n-1} bAct_i.mAct_i.wAct_i}{n}$$

wherein, bActi is a specific activity involving hand, $mAct_i$ is a magnitude of activity involving hand and wActi is a weightage assigned to each hand activity.

5. The method as claimed in claim 2, wherein acquiring heart rate variability using PPG sensors to sense physiological abnormality (vPpm) due to alcohol intoxication is calculated by an equation:

$$vPpm = \frac{\sum_{i=0}^{n-1} bPpm_i.mPpm_i.wPpm_i}{n}$$

wherein, $bPpm_i$ is an array holding all physiological indicators, mPpmi is an array of magnitudes of all markers, and $wPpm_i$ indicate weights for each marker in overall computation.

6. The method as claimed in claim 2, wherein the method derives a correlation of the GPS location based on vGt, vAct and vPpm to derive a correlation score (vLoc) as per equation:

$$vLoc = \frac{\sum_{i=0}^{n-1} bLoc_i.mLoc_i.wLoc_i}{n}$$

wherein, $bLoc_i$ is the flag indicating a location publicly known to serve the alcohol or a place known to be frequently visited by the person, mLoci indicates the magnitude of flags indicated, and wLoci indicate weights assigned for each flag.

7. The method as claimed in claim 2, wherein $R_{sti}$, an abnormality in responsiveness to stimuli is calculated as per the equation:

$$vResponsivenessToStimuli\ (R_{sti}) = \frac{vComprehension\ +\ vReflex}{2}$$

wherein, vComprehension is the score for comprehensive ability and vReflex is the score for reflexive ability.

8. The method as claimed in claim 1, wherein the alerts are sent to the subject or to the emergency contact by a plurality of ways wherein the plurality of ways comprising (a) alerting the person when the intoxication confidence score found within a threshold value; (b) alerting the emergency contact when the intoxication confidence score found to exceed a threshold value; (c) alerting the emergency contact for SoS when the intoxication confidence score is found to exceed the threshold value and an individual scores of the alertness detection module and the physiology assessment module shows abrupt pattern.

9. A system, comprising:

a memory storing instructions;
one or more communication interfaces; and
one or more hardware processors coupled to the memory via the one or more communication interfaces, wherein the one or more hardware processors are configured by the instructions to:

receive a global positioning system (GPS) location of a subject from a mobile device carried by the subject;
acquire motion sensing data of the subject comprising gait pattern, activity sensing and hand gestures using inertial motion sensors of the mobile device and a wearable device worn by the subject;
acquire cognitive ability sensing data of the subject from the mobile device based on a response of the subject to a plurality of activities performed on the mobile device, wherein the plurality of activities comprising

(i) interacting with an application installed on the mobile device, personalized for the subject,
(ii) performing gaze tracking by utilizing a camera of the mobile device, and
(iii) performing voice and speech analysis by utilizing a microphone of the mobile device, wherein the cognitive ability sensing data is processed to obtain an attentiveness score;

acquire physiology sensing data from a plurality of sensors on the wearable device and the mobile device, the physiological sensing data comprising (i) body temperature via thermistors, (ii) sweat analysis based on sweat sensing via transdermal sensors, (iii) heart rate variability via Photoplethysmograph (PPG) sensors, wherein the physiology sensing data is proceesed to obtain a physiology score;
obtain a correlation score using a location sensing module wherein the correlation score is derived by a time lapse buffer based on presence of the subject on a given GPS location and episodes of alcohol consumption;
aggregate the alertness score, the attentiveness score, the physiology score, and the correlation score to obtain an intoxication confidence score of the person; and

alert one of the subject and an emergency contact about the intoxication state of the person in accordance with the intoxication confidence score.

10. The system as claimed in claim 9, wherein the intoxication confidence score to sense the state of alcohol intoxication of the subject is calculated by an equation:

$$vIntoxication = \left(\frac{vGt + vPpm + vAct + vLoc}{4}\right) * R_{sti}^{-1}$$

wherein *vGt* is an abnormality in the gait pattern, *vAct* is an abnormality in the hand gesture, *vPpm* is a physiological abnormality, *vLoc* is a correlation score and $R_{sti}$ is an abnormality in responsiveness to stimuli.

11. The system as claimed in claim 10, wherein *vGt,* the abnormality in the gait pattern due to alcohol intoxication is calculated by an equation:

$$vGt = bGt.mGt.wGt$$

wherein, bGt indicates gait pattern, mGt is a magnitude of abnormality and wGt is the weightage assigned to gait sensing in the overall computation, and wherein *vAct,* the abnormality in the hand gestures due to alcohol intoxication is calculated by an equation:

$$vAct = \frac{\sum_{i=0}^{n-1} bAct_i . mAct_i . wAct_i}{n}$$

*vAct* indicates confidence in performing hand gesture related activities, bActi is a specific activity involving hand, mActi is a magnitude of activity involving hand and $wAct_i$ is a weightage assigned to each hand activity.

12. The system as claimed in claim 10, wherein acquiring the heart rate variability using PPG sensors to sense physiological abnormality (vPpm) due to alcohol intoxication is calculated by an equation:

$$vPpm = \frac{\sum_{i=0}^{n-1} bPpm_i . mPpm_i . wPpm_i}{n}$$

wherein, bPpmi is an array holding all physiological indicators, mPpmi is an array of magnitudes of all markers, and $wPpm_i$ indicate weights for each marker in overall computation, and wherein the method derives a correlation of the GPS location based on vGt, vAct and vPpm to derive a correlation score *(vLoc)* as per equation:

$$vLoc = \frac{\sum_{i=0}^{n-1} bLoc_i . mLoc_i . wLoc_i}{n}$$

wherein, bLoci is the flag indicating a location publicly known to serve the alcohol or a place known to be frequently visited by the person, mLoci indicates the magnitude of flags indicated, and $wLoc_i$ indicate weights assigned for each flag, and wherein $R_{sti}$ the abnormality in responsiveness to stimuli is calculated as per equation:

$$vResponsivenessToStimuli \ (R_{sti}) = \frac{vComprehension \ + \ vReflex}{2}$$

wherein, vComprehension is the score for comprehensive ability and vReflex is the score for reflexive ability.

13. The system as claimed in claim 9, wherein the alerts are sent to the subject or to the emergency contact by a plurality of ways wherein the plurality of ways comprising (a) alerting the person when the intoxication confidence score found within a threshold value; (b) alerting the emergency contact when the intoxication confidence score found to exceed a threshold value; (c) alerting the emergency contact for SoS when the intoxication confidence score is found to exceed the threshold value and an individual scores of the alertness detection module and the physiology assessment module shows abrupt pattern.

14. One or more non-transitory machine-readable information storage mediums comprising one or more instructions which when executed by one or more hardware processors cause:

receiving by a cloud server, a global positioning system (GPS) location of a subject from a mobile device carried by the subject;
acquiring motion sensing data of the subject comprising gait pattern, activity sensing and hand gestures using inertial motion sensors of the mobile device and a wearable device worn by the subject;
acquiring cognitive ability sensing data of the subject from the mobile device based on a response of the subject to a plurality of activities performed on the mobile device, wherein the plurality of activities comprising

(i) interacting with an application installed on the mobile device, personalized for the subject,
(ii) performing gaze tracking by utilizing a camera of the mobile device, and
(iii) performing voice and speech analysis by utilizing a microphone of the mobile device, wherein the cognitive ability sensing data is processed to obtain an attentiveness score;

acquiring physiology sensing data from a plurality of sensors on the wearable device and the mobile device, the physiological sensing data comprising (i) body temperature via thermistors, (ii) sweat analysis based on sweat sensing via transdermal sensors, (iii) heart rate variability via Photoplethysmograph (PPG) sensors, wherein the physiology sensing data is proceesed to obtain a physiology score;

obtaining a correlation score using a location sensing module wherein the correlation score is derived by a time lapse buffer based on presence of the subject on a given GPS location and episodes of alcohol consumption;

aggregating the alertness score, the attentiveness score, the physiology score, and the correlation score to obtain an intoxication confidence score of the person; and

alerting one of the subject and an emergency contact about the intoxication state of the person in accordance with the intoxication confidence score.

15. The one or more non-transitory machine-readable information storage mediums of claim 14, wherein the intoxication confidence score to sense the state of alcohol intoxication of the subject is calculated by an equation:

$$vIntoxication = \left( \frac{vGt + vPpm + vAct + vLoc}{4} \right) * R_{sti}^{-1}$$

wherein $vGt$ is an abnormality in the gait pattern, $vAct$ is an abnormality in the hand gesture, $vPpm$ is a physiological abnormality, $vLoc$ is a correlation score and $R_{sti}$ is an abnormality in responsiveness to stimuli.

System **100**

IoT enabled
wearable device
**112**

Processors(s) **104**    I/O Interface(s) **106**

Database **108**    Cloud server 116

Smartphone 114

Memory **102**

INTOXICATION DETECTION
MODEL 110

Alertness detection module 110A

Attentiveness detection module
110D

Physiology assessment module
110C

Location sensing module 110D

Fuse and analyze module 110E

FIG. 1

FIG. 2

100

| Alertness detection module 110A | → | Attentiveness detection module 110B | → | Physiology assessment module 110C |

302 GAIT assessment

304 Fall prediction & detection

306 Activity tracking & detection

308 Hand Gesture

310 Motion tracking

312 Interaction with the Smartphone application

314

316 Gaze tracking

Voice & Speech analysis

318 Heart rate & breathing pattern

320 Body temperature

322 Sweat analysis

Location sensing module 110D

Fuse & analyze module 110E

324 Subject alert generation

326 Phase-I SoS trigger

328 Phase-II SoS trigger

FIG. 3

23

400

| obtaining GPS location of the subject possessing the wearable device and the smart phone | 402 |

| obtaining an alertness score by combining the output of sub-modules performing GAIT assessment, fall prediction & detection, activity tracking & detection, hand gesture and motion tracking | 404 |

| obtaining an attentiveness score by combining the output of sub-modules performing gaze tracking, speech & voice analysis and the interaction of the subject with the privileged smart phone application | 406 |

| obtaining physiology score by combining the output of sub-module performing heart-rate and breathing pattern analysis, body temperature and sweat analysis | 408 |

( A )

FIG. 4A

A

obtaining correlation score of the subject present on given GPS location and alcohol
consumption based on location stamp data using time lapse buffer — 410

aggregating alertness score, attentiveness score, physiology score and correlation
score using fuse and analyze module to obtain intoxication confidence score — 412

alerting the subject about intoxication state from the plurality of possible ways:

(i) alerting the subject about level of alcohol consumption using the wearable device
and the smart phone when the intoxication score found within the threshold,

(ii) alerting the emergency contact about subject's level of alcohol consumption
using the smart phone when the intoxication score exceeds the threshold, or

(iii) sending SoS for panic situation to the emergency contact when the intoxication
score exceeds the threshold and individual scores of alertness detection module and
physiology assessment module shows abrupt pattern

— 414

FIG. 4B

Start: On-boarding

502 — Obtain consent for tracking

504 — Provide Emergency contacts → A

506 — Pair smart wearable device for activity tracking

508 — App customization

Yes → Enable passive tracking in background

No → Schedule a timer to auto launch gamified operation

Timer triggered — 510

512 — Access user by gamified application

B

Stop: On-boarding done

FIG. 5

A

Emergency contact verification

602 — Send verification as well as consent
link to emergency contact

604

Verified and consent
provided

No

608

Discard contact

Yes

606

Add contact

Ask more contacts

Run confirmation

FIG. 6

702 → **Start assessment**

**Randomly choose assessment criteria**

704

706

708

| **Wearable-only assessment** | **wearable+phone assessment** | **Phone only assessment** |

C      D      E

**Example:** User is asked to walk/brisk-walk/ sit/climb-stairs etc. User's patterns are analyzed and scored

**Example:** User is shown 3+/-2 patterns on the phone screen. Then user is asked to make the pattern in "air" using the arm on which user is wearing smart watch. All patterns created by the user are assessed and scored based on correctness.

**Example:** User is shown 3+/-2 interactive games. 1. A moving "dot" is shown on the phone screen and user asked to track the dots movement with his/her eyes. Gaze trajectory is analyzed and recorded. 2. A 3x3 pattern is drawn on the screen and user is asked to repeat the pattern by swiping finger on the touch-screen. Swipe gestures are analyzed and recorded.

**Tune user's model**

**Stop: return**

FIG. 7

FIG. 8

Start: Phase II sensing

↓

Wait for user to use the phone

↓

Has user picked phone? — 902

↓ yes

Randomly choose assessment criteria

↓

Has user accomplished task satisfactorily? — 904

↓

Evaluate how poorly task was performed

↓

Record performance score in TimeLapse Buffer — 906

↓

Differ test for some interval — 908

↓

Allow user to use phone — 910

FIG. 9

1002

V

Evaluate how poorly task is performed

1004

$t_{1\%} < vIntoxication_n < t_{2\%}$

Alert user for over consumption

1006

$t_{2\%} < vIntoxication_n < t_{3\%}$

Alert user for over consumption

Alert emergency contacts

1008

$vIntoxication_n > t_{3\%}$

Evaluate GAIT abnormality and fall probability

If both GAIT abnormality and fall probability are high

Send SoS Panic emergency contact

Evaluate heart rate abnormality

If heart rate abnormality is high

Return

FIG. 10

Europäisches
Patentamt

European
Patent Office

Office européen
des brevets

## EUROPEAN SEARCH REPORT

**Application Number**

EP 24 21 3246

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/190188 A1 (NOTHACKER KEITH HARRY [US] ET AL) 22 June 2023 (2023-06-22) * paragraphs [0002], [0018], [0033] - [0080], [0100], [0110], [0155], [0156], [0160], [0161] * * figures 1-3,10 * | 1-15 | INV. A61B5/0205 A61B5/024 A61B5/11 A61B5/145 A61B5/16 A61B5/18 A61B5/00 |
| A | US 2021/113153 A1 (NOTHACKER KEITH HARRY [US] ET AL) 22 April 2021 (2021-04-22) * paragraphs [0024] - [0032], [0044], [0054], [0059] - [0076], [0088] - [0143] * | 1-15 | |
| A | HSIN-LIU (CINDY) KAO ET AL: "Phone-based gait analysis to detect alcohol usage", UBIQUITOUS COMPUTING, ACM, 2 PENN PLAZA, SUITE 701 NEW YORK NY 10121-0701 USA, 5 September 2012 (2012-09-05), pages 661-662, XP058042271, DOI: 10.1145/2370216.2370354 ISBN: 978-1-4503-1224-0 * the whole document * | 1,2,6,9, 10,12, 14,15 | |

**TECHNICAL FIELDS SEARCHED (IPC)**

A61B

The present search report has been drawn up for all claims

| Place of search | Date of completion of the search | Examiner |
|---|---|---|
| The Hague | 9 April 2025 | Van der Haegen, D |

**EP 4 566 520 A1**

## ANNEX TO THE EUROPEAN SEARCH REPORT
## ON EUROPEAN PATENT APPLICATION NO.

EP 24 21 3246

09-04-2025

| Patent document cited in search report | Publication date | Patent family member(s) | Publication date |
|---|---|---|---|
| US 2023190188 A1 | 22-06-2023 | NONE | |
| US 2021113153 A1 | 22-04-2021 | US 2021113153 A1 | 22-04-2021 |
| | | US 2022287641 A1 | 15-09-2022 |
| | | US 2024090839 A1 | 21-03-2024 |
| | | US 2025032048 A1 | 30-01-2025 |

EPO FORM P0459

**EP 4 566 520 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321083835